Europäisches Patentamt

European Patent Office

Office européen des brevets

(19)

(11) Veröffentlichungsnummer: **0 247 439**
B1

(12) **EUROPÄISCHE PATENTSCHRIFT**

(45) Veröffentlichungstag der Patentschrift:
28.02.90

(21) Anmeldenummer: 87106932.4

(22) Anmeldetag: 13.05.87

(51) Int. Cl.⁴: **G01N 33/48**, G01N 35/00

(54) System zur Bestimmung der Konzentration von Bestandteilen von Körperflüssigkeiten.

(30) Priorität: 22.05.86 DE 3617161

(43) Veröffentlichungstag der Anmeldung:
02.12.87 Patentblatt 87/49

(45) Bekanntmachung des Hinweises auf die Patenterteilung:
28.02.90 Patentblatt 90/9

(84) Benannte Vertragsstaaten:
AT BE CH DE ES FR GB GR IT LI LU NL SE

(56) Entgegenhaltungen:
EP-A- 0 073 056
EP-A- 0 132 790
DE-A- 2 542 674

G.I.T. FACHZEITSCHRIFT FÜR DAS LABORATORIUM,
Band 15, Nr. 7, Juli 1971, Seiten 741-746; E. BLATT:
"Datenverarbeitung im klinisch-chemischen Labor"

(73) Patentinhaber: BOEHRINGER MANNHEIM GMBH,
Sandhofer Strasse 116, D-6800 Mannheim 31(DE)

(72) Erfinder: Herpichböhm, Bernd, Dr. rer. nat.,
Eugen-Richter-Strasse 7, D-6800 Mannheim 24(DE)

## Beschreibung

Die vorliegende Erfindung betrifft ein System nach dem Oberbegriff des Anspruchs 1.

In neuerer Zeit werden zunehmend Testträger, beispielsweise in Form von Teststreifen oder in Form von flachen, näherungsweise quadratischen Plättchen zur Analyse von Körperflüssigkeiten, insbesondere von Urin und Blut eingesetzt. Mit diesen Testträgern können analytische Bestimmungen, insbesondere für medizinische Zwecke, besonders einfach durchgeführt werden. Zunächst werden die Testträger mit der Probe in Kontakt gebracht, was bei der Untersuchung von Urin im allgemeinen durch Eintauchen eines streifenförmigen Testträgers geschieht, während zur Untersuchung von Blut üblicherweise ein Blutstropfen auf das Testfeld des Testträgers aufgebracht und nach dem Vollsaugen des Testfeldes abgewischt oder abgewaschen wird. In jedem Fall enthält das Testfeld, das den für die analytische Bestimmung entscheidenden Teil des Testträgers darstellt, Reagenzien, die mit den Inhaltsstoffen der aufgebrachten Körperflüssigkeit reagieren.

Zur Auswertung der Reaktion wird der Testträger in ein entsprechendes Auswertegerät eingelegt. Dort wird, üblicherweise zu einem bestimmten Zeitpunkt nach Aufbringen der Probe, ein physikalischer Meßwert gemessen, der als Maß für die Konzentration eines bestimmten Bestandteiles der Körperflüssigkeit dient. Bei der Analyse von Körperflüssigkeiten wird weit überwiegend eine Reaktion verwendet, die zu einer für die durchzuführende Analyse charakteristischen Farbveränderung führt. In diesem Fall wird üblicherweise das diffuse Reflektionsvermögen des Testfeldes bei einer bestimmten Wellenlänge als physikalischer Meßwert bestimmt. Die Erfindung richtet sich insbesondere auf solche photometrisch auszuwertenden Testträger. Bei einem anderen bekannten Testträgertyp wird eine elektrochemische Reaktion benutzt. In diesem Fall wird eine Spannung oder eine Stromstärke als für die Analyse charakteristischer physikalischer Meßwert bestimmt.

Die Testträger sind üblicherweise spezifisch für eine bestimmte Analyse geeignet, d.h. zur Feststellung der Konzentration eines bestimmten Bestandteils einer Körperflüssigkeit, eines sogenannten Parameters, wird vom Hersteller ein bestimmter Testträgertyp angeboten. Zur Auswertung der Testträger werden sowohl Geräte verwendet, die speziell auf einen derartigen Testträgertyp ausgerichtet sind (Einparameter-Geräte) als auch solche, die mehrere verschiedene Testträger ausmessen können (Mehrparameter-Geräte).

An die Testträger und die zugehörigen Auswertegeräte, die gemeinsam ein System bilden, werden sehr hohe Genauigkeitsanforderungen gestellt. Üblicherweise wird eine große Anzahl von Testträgern unter gleichbleibenden Bedingungen gemeinsam hergestellt. Beispielsweise bei photometrisch auswertbaren Tests wird für eine Herstellungscharge eine bestimmte Trägermatrix für die Testfelder und ein einziger Ansatz von Reagenzien verwendet. Die Testträger einer solchen Herstellungscharge stimmen daher in ihren Eigenschaften nahezu vollständig überein. Andererseits weichen die Testträger verschiedener Herstellungschargen in ihrer Auswertekurve, d.h. in der Relation zwischen dem vom Gerät bestimmten physikalischen Meßwert, insbesondere dem diffusen Reflexionsvermögen des Testfeldes, und der zu bestimmenden Konzentration des gesuchten Parameters voneinander ab. Diese Abweichungen sind so groß, daß die in der Medizin gestellten hohen Anforderungen für quantitative Bestimmungen nicht erfüllt werden können, wenn die Unterschiede zwischen den verschiedenen Herstellungschargen vernachläßigt werden.

Die gewünschte Genauigkeit könnte prinzipiell durch Eichung mit Standardlösungen vor jedem Gebrauch eines Testträgers erreicht werden. Dies erschwert jedoch die Handhabung des analytischen Systems.

Es ist daher bereits in der Vergangenheit vorgeschlagen worden, dem Auswertegerät die Information über die chargenspezifische Auswertekurve der jeweiligen Testträgercharge in geeigneter Weise zu übermitteln. So ist in der europäischen Patentanmeldung mit der Publikationsnummer 73 056 beschrieben, daß die einzelnen Testträger mit einem Strichcode versehen werden können, der Informationen über die chargenspezifische Auswertekurve enthält. Das zugehörige Auswertegerät enthält einen Strichcode-Leser zur Erfassung der Information.

Die europäische Patentanmeldung mit der Publikationsnummer 132 790 beschreibt streifenförmige Testträger, die eine Magnetschicht aufweisen, welche ebenfalls zur Speicherung von Informationen geeignet ist. Diese werden vom Hersteller der Testträger in die Magnetschicht eingebracht und im Auswertegerät mittels eines entsprechenden Magnetlesekopfes ausgelesen.

Diese beiden bekannten Verfahren erlauben eine besonders bequeme Handhabung, weil die Geräte die notwendigen Informationen jeweils automatisch mitgeteilt bekommen, ohne daß der Benutzer irgendwelche Handhabungsschritte hierzu unternehmen muß. Diesem Vorteil steht jedoch ein erheblicher Aufwand gegenüber. Die der Herstellung des Testträgers muß jedem einzelnen Testträger die Information übermittelt werden und es müssen spezielle, auf den jeweiligen Gerätetyp abgestimmte Testträger produziert werden. Im Gerät muß jeweils eine entsprechende Leseeinrichtung vorhanden sein, die die Kosten des Gerätes wesentlich erhöht.

Für einfacherer Geräte, insbesondere zur Bestimmung von Blutglukosewerten für Diabetiker, ist es daher weitgehend üblich, die chargenspezifischen Variationen der Testträger zu vernachlässigen. Dies führt jedoch zu medizinisch nicht tolerierbaren Ungenauigkeiten. Bei einem anderen bekannten Blutglukose-Bestimmungsgerät wird den Teststreifenpackungen jeweils auf einem von den Testträgern getrennten Film die chargenspezifische Auswertekurve in Form eines Strichcode mitgegeben. Dadurch werden zwar die Aufwendungen bei der Herstellung der Testträger reduziert, das Gerät muß aber auch in diesem Fall einen Codeleser haben und wird dadurch erheblich verteuert.

Aufgabe der vorliegenden Erfindung ist es, ein System der eingangs bezeichneten Art dahingehend zu verbessern, daß die chargenspezifische Auswertekurve berücksichtigt wird, so daß eine den medizinischen Anforderungen voll genügende Genauigkeit erreicht wird und gleichzeitig die Herstellungskosten sowohl der Testträger als auch des Auswertegerätes möglichst günstig sind.

Diese Aufgabe wird durch die in den Patentansprüchen definierte Erfindung gelöst.

Aufgrund der Erfindung kann das Gerät sehr einfach auf die Auswertung der jeweiligen Teststreifencharge eingerichtet werden. Auf der Packung der zugehörigen Testträger wird beispielsweise eine Ziffern- oder Zeichenfolge aufgedruckt, wobei bevorzugt die Gesamtheit der Berechnungsstützpunkte in mehrere, vorzugsweise gleich große Teilmengen, die als Sätze bezeichnet werden, unterteilt ist und die Anzahl der Zeichen der Anzahl der Sätze von Berechnungsstützpunkten entspricht. Diese wird an dem Gerät mittels einer geeigneten Einstelleinrichtung eingestellt. In Frage kommen beispielsweise Dezimalschalter mit einer der Anzahl der Berechnungsstützpunkt-Sätze entsprechenden Anzahl von Stellen, die sich auf jeweils beispielsweise 10 verschiedene Werte einstellen lassen. Eine andere Möglichkeit besteht darin, entsprechende Tasten vorzusehen, die zur Codeeingabe benutzt werden können, wie dies weiter unten näher beschrieben wird. In jedem Fall muß der Benutzer des Geräts lediglich einmal bei Anbrechen einer neuen Packung mit Testträgern den Code der Charge überprüfen und ggf. entsprechend die Einstellung der Einstelleinrichtung an dem Gerät ändern. Aufgrund dieser wenigen Einstellwerte kann das Gerät aufgrund des neuartigen Verfahrens,nach welchem Mikroprozessor aus den physikalischen Meßwerten die Konzentration berechnet, diese Konzentration mit einer Genauigkeit bestimmen, wie sie bisher bei derartig einfachen Geräten nicht möglich war.

Die Tatsache, daß das erfindungsgemäße Auswertegerät keinen Codeleser benötigt, spart nicht nur Kosten, sondern erlaubt auch eine besonders kleine Bauweise. Dies ist auch medizinisch von Bedeutung, weil gerade Diabetiker ihren Blutzuckerspiegel möglichst unter normalen Lebensbedingungen messen sollen und deswegen das Meßgerät auch außer Haus benutzen. Die Akzeptanz eines Systems zur Blutzuckerbestimmung ist deswegen umso besser, je kleiner und leichter transportabel das Gerät ist.

Allgemein gesprochen erlaubt die Erfindung eine erhebliche Reduzierung der Informationsmenge, die zur exakten chargenspezifischen Auswertung von Testträgern verarbeitet werden muß. Dadurch wird nicht nur die Übermittlung der Auswertekurve an das Gerät vereinfacht, sondern auch die Verarbeitung der Information im Gerät selbst. Infolgedessen kann häufig ein einfacher 4-bit-Mikrroprozessor statt eines erheblich aufwendigeren 8-bit-Mikrropozessors verwendet werden. Auch hierdurch ergibt sich eine erhebliche Einsparung an Kosten und Baugröße.

Besonders gute Ergebnisse werden erzielt, wenn in dem Gerät eine Standardauswertekurve abgespeichert ist, die einen ähnlichen Verlauf wie die Auswertekurven der von dem Gerät auszuwertenden Testträger hat, wobei in der Umgebung der Wendepunkte der Kurven sowie der Kurvenenden Standardstützpunkte auf der Standardkurve definiert und die Sätze der Berechnungsstützpunkte in der Umgebung der Standardstützpunkte vorgesehen sind. Dabei wird insbesondere die Relation des Abstandes der dem jeweiligen Meßwert nächstliegenden Berechnungsstützpunkte einerseits und des Abstandes der nächstliegenden Standardstützpunkte andererseits in die Berechnung miteinbezogen.

Die Erfindung wird im folgenden anhand eines in den Figuren dargestellten Ausführungsbeispiels näher erläutert. Es zeigen:

Figur 1 ein Blockschaltbild eines erfindungsgemäßen Auswertegerätes für Testträger

Figur 2 eine graphische Darstellung zweier chargenspezifischen Auswertekurven und einer Standardauswertekurve

Figur 3 einen Ausschnitt einer chargenspezifischen Auswertekurve und einer Standardauswertekurve mit gegenüber Figur 2 abgewandelter Lage der Berechnungsstützpunkte-Wertepaare.

Figuren 4 und 5 Beispiele für die Aufbringung der sichtbaren Zeichen auf dem Testträger bzw. dessen Verpackung oder Verpackungsbeilage.

Das Testfeld wird von dem Licht eines Lichtsenders (16) beleuchtet. Das von der Oberfläche des Testfeldes (14) diffus reflektierte Licht wird von einer Meßeinrichtung (18) erfaßt. Die Meßeinrichtung umfaßt üblicherweise einen lichtempfindlichen Empfänger, beispielsweise eine Fotodiode, einen Verstärker und einen Analog-Digital-Wandler. Geeignete Meßeinrichtungen sind bekannt.

Zentrales Element des Geräts ist ein Prozessor (20) mit zugehörigem, nicht gesondert dargestelltem Speicher. Er dient dazu, die verschiedenen Gerätefunktionen zu steuern und die von der Meßeinrichtung (18) gelieferten physikalischen Meßwerte in die gesuchten Konzentrationswerte umzurechnen. Die so errechneten Werte werden mittels einer Anzeigensteuerung (22) auf einer Anzeige (24) angezeigt. Als Anzeige wird üblicherweise ein digitales Display verwendet.

Um aus den physikalischen Meßwerten die entsprechenden Konzentrationswerte berechnen zu können, benötigt der Prozessor (20) Informationen über die chargenspezifische Auswertekurve. Diese werden ihm im dargestellten Ausführungsbeispiel der Erfindung über drei Tasten (26, 28, 30) eingegeben. Die mit Hilfe der Tasten gewählten Werte werden auf dem Display (24) angezeigt. Wenn die Anzeigen des Displays den beispielsweise auf der Testträgerpackung angegebenen Code richtig wiedergeben, wird dem Prozessor (20) der Befehl gegeben, den auf dem Display angezeigten Code zu übernehmen und abzuspeichern.

Das Display kann beispielsweise als übliches 7-Segment-Display ausgebildet sein, mit dem neben den zehn Ziffern des Dezimalsystems auch eine Reihe von Zeichen angezeigt werden können. In einer praktisch bevorzugten Ausführungsform kann jede der Stellen des Display 16 verschiedene Zeichen anzeigen, so daß mit Hilfe der Tasten 26 bis 28 für jede Stelle des Codes 16 verschiedene diskrete Werte angezeigt und an den Prozessor (20) übermittelt werden können. Die Zahl 16 ist besonders bevorzugt, weil bis zu 16 verschiedene Werte leicht mit Hilfe eines 4-Bit-Wortes innerhalb des Prozessors codiert und verarbeitet werden können. Je nach den Anforderungen kann aber auch eine andere Anzahl diskreter Einstellwerte zur Anwendung kommen.

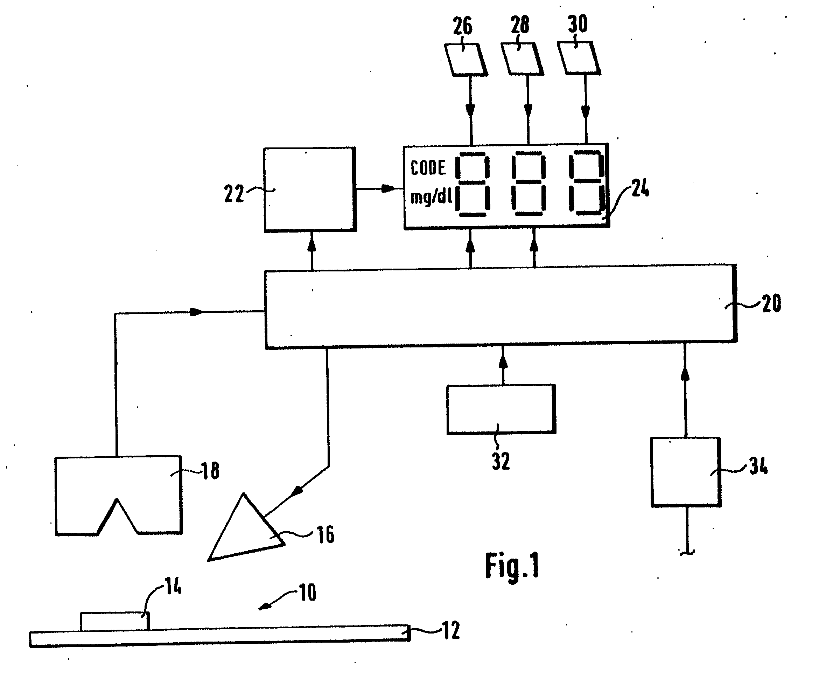

In Figur 1 erkennt man weiterhin noch einen Timer (32) zur Steuerung des Mikroprozessors und ein Netzteil (34) zur Energieversorgung des Systems. Andere Elemente, wie beispielsweise Elemente zur Kontrolle der Betriebsspannung und dergleichen, sind wie erwähnt der Übersichtlichkeit halber nicht dargestellt.

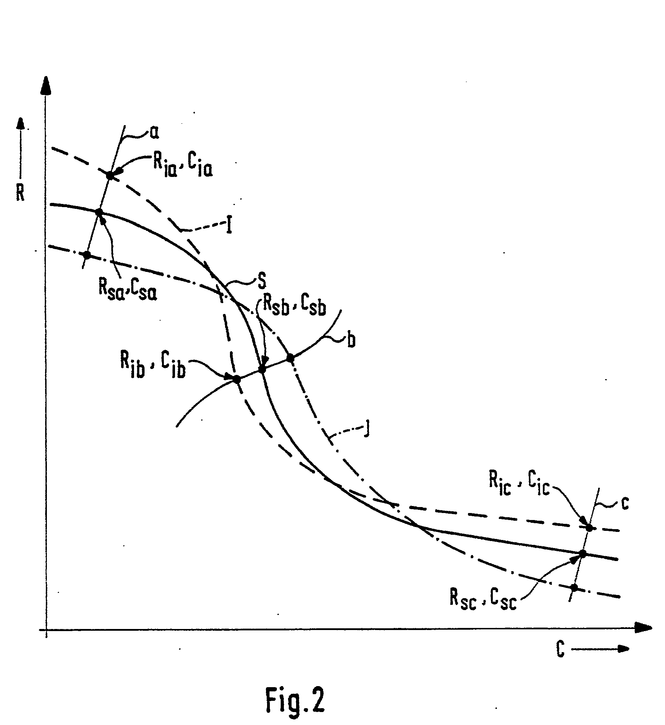

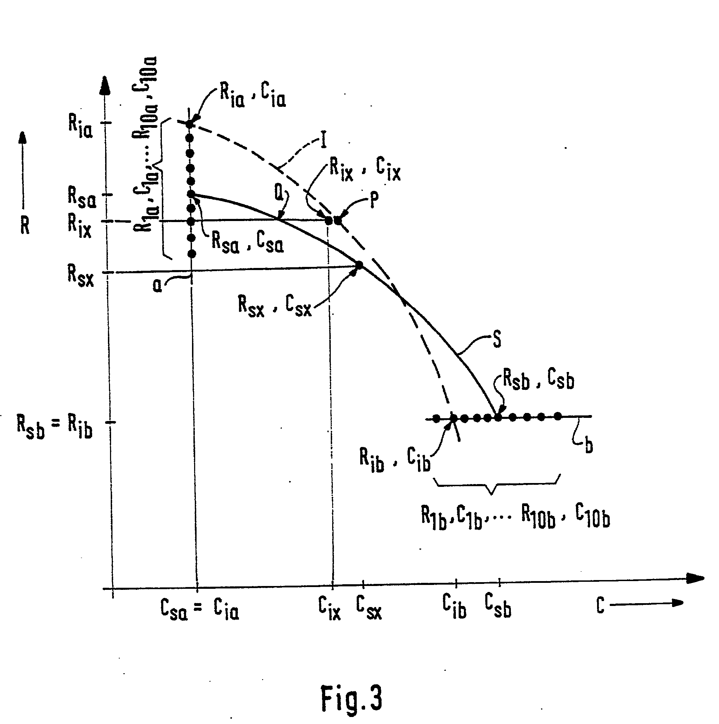

Die Berechnung der Konzentration aus den physikalischen Meßwerten in dem Prozessor (20) erfolgt mit Hilfe des beispielsweise über die Tasten 26, 28, 30 und das Display (24) eingegebenen aus nur wenigen Stellen mit jeweils einer geringen Anzahl diskreter Werte bestehenden Code. Dieses Verfahren wird im folgenden anhand der Figuren 2 und 3 näher erläutert. In Figur 2 sind drei Kurven S, I und J eingezeichnet. Die gestrichelte Kurve I zeigt den Verlauf einer beispielhaften Auswertekurve für einen optischen Test zur Bestimmung der Glucose im Blut. Auf der Ordinate ist das diffuse Reflektionsvermögen R bei einer bestimmten durch das optische System des Geräts festgelegten Meßwellenlänge aufgetragen. Auf der Abszissenachse ist die entsprechende Konzentration des zu bestimmenden Parameters, hier also der Glucose, aufgetragen. Man erkennt bei der Kurve I, daß aufgrund der charakteristischen Eigenschaften der Testfelder des analytischen Testträgers das diffuse Reflexionsvermögen R bei niedrigen Konzentrationswerten hoch ist. Im mittleren Konzentrationsbereich nimmt es mit einer von rechts oben gesehen konvexen Krümmung zunehmend ab. Der Kurvenverlauf geht dann nach Durchlaufen eines Wendepunktes in eine konkave Krümmung über. Das diffuse Reflexionsvermögen R sinkt in diesem Bereich mit zunehmender Konzentration C weniger stark und verändert sich schließlich bei hohen Konzentrationen nur noch wenig.

Der allgemeine Verlauf von Auswertekurven von Testträgern ist von den gewählten Reagenzien und sonstigen Eigenschaften der Testträger abhängig und daher von Test zu Test verschieden. Ein Verlauf der hier dargestellten Art ist jedoch für viele Fälle typisch. Er hat den Vorteil, daß im mittleren Bereich eine hohe Abhängigkeit der Remission von der Konzentration gegeben ist. Dies ist günstig für eine hohe Meßgenauigkeit.

Wie erwähnt, sind die Auswertekurven für verschiedene Testträger einer Herstellungscharge nicht gleich. Beispielhaft ist in Figur 2 eine weitere Auswertekurve strichpunktiert eingezeichnet, die mit J bezeichnet ist. Man erkennt, daß diese Kurve erheblich verschieden ist von der Auswertekurve I. Sie stimmt jedoch in einigen grundsätzlichen Eigenschaften mit dieser überein. Insbesondere hat auch sie einen zunächst konkaven und dann nach einem Wendepunkt einen konvexen Verlauf. Weitere Auswertekurven sind der Übersichtlichkeit halber nicht dargestellt. Es sei jedoch betont, daß jede Charge eine andere Auswertekurve hat, so daß es eine unendlich große Vielzahl von möglichen Auswertekurven gibt, die im Rahmen der Erfindung in guter Näherung berücksichtigt werden können.

Wesentlich für die vorliegende Erfindung ist nun, daß eine Standardauswertekurve festgelegt und in dem Speicher des Auswertegeräts abgespeichert wird, deren Verlauf dem der Auswertekurven in dem im folgenden definierten Sinne ähnlich ist. Eine derartige Standardauswertekurve ist in Figur 2 durchgezogen eingezeichnet und mit S bezeichnet.

Die Standardauswertekurve soll zunächst im Wertebereich der Auswertekurven verlaufen. Damit ist gemeint, daß die Standardauswertekurve zumindest in dem gewünschten Konzentrationsbereich innerhalb der Extremwerte des physikalischen Meßwertes der in der Praxis vorkommenden Auswertekurven der Testträger liegt. Wie in Figur 2 dargestellt ist, werden diese Extremwerte nicht etwa durchgängig von den gleichen Auswertekurven markiert. In der dargestellten Ausführungsform hat die Kurve I zum Beispiel im Bereich niedriger und hoher Konzentrationen besonders hohe R-Werte und die Kurve J besonders niedrige R-Werte. Im Bereich mittlerer Konzentrationen ist es gerade umgekehrt. Die Standardauswertekurve S soll irgendwie im Bereich der Auswertekurven liegen. In vielen Fällen ist es besonders günstig, wenn sie etwa im mittleren Wertebereich der Auswertekurven, also etwa im Zentrum der durch eine Vielzahl von Vorversuchen ermittelten Auswertekurven verschiedener Testträgerchargen verläuft.

Weiter soll die Standardauswertekurve eine konvexe bzw. konkave Krümmung im wesentlichen in den gleichen Abschnitten von Werten des diffusen Reflexionsvermögens aufweisen wie die Auswertekurven. Quantitativ läßt sich sagen, daß der Wendepunkt zwischen einem konkav und einem konvex gekrümmten Bereich der Standardkurve bei einem solchen Wert des diffusen Reflexionsvermögens liegen sollte, daß die den Wendepunkten der möglichen Auswertekurven der Testträgerchargen entsprechenden Werte des diffusen Reflexionsvermögens sich um höchstens 20 %, bevorzugt höchstens 10 % von diesem unterscheiden.

Wie aus Figur 2 zu erkennen ist, stimmen die Konzentrationsbereiche, in denen die Kurven I und J die gleiche Krümmung aufweisen, nicht gut überein. Insbesondere liegt der Wendepunkt nicht beim gleichen Konzentrationswert. Es ist deshalb nicht möglich und auch nicht notwendig, daß die Standardauswertekurve in den genau gleichen Abschnitten von Konzentrationswerten die gleiche Krümmung aufweist wie die Auswertekurven. Es genügt, wenn diese weitgehend übereinstimmen, so daß in dem weiter unten näher beschriebenen Iterationsverfahren ausreichend exakte Werte des Meßergebnisses erreicht werden.

Die Standardkurve kann z. B. in Form eines Polynoms ausgedrückt werden, dessen Koeffizienten abgespeichert werden. Bevorzugt wird sie als Polygon dargestellt, d. h. es werden Punkte, die auf dem Kurvenverlauf liegen, abgespeichert und zwischen den Punkten wird linear interpoliert. Selbstverständlich kann die Standardkurve auch in einer transformierten Form (z. B. linear transformiert) abgespeichert werden. In diesem Fall muß die Transformation bei den folgenden Berechnungen berücksichtigt werden.

Weiter ist für die Erfindung wesentlich, daß auf der Standardkurve in der Nähe jedes Wendepunktes zwischen einem konkav und einem konvex gekrümmten Abschnitt ein Punkt definiert und im Speicher abgespeichert ist, der als Standardstützpunkt bezeichnet wird. Er ist in Figur 2 mit $R_{sb}$, $C_{sb}$ bezeichnet. Der Begriff "in der Nähe" ist hier analog zu dem Begriff "im wesentlichen" im vorhergehenden Absatz zu verstehen, d. h. der Konzentrationswert des Standardstützpunktes darf sich nicht so sehr vom Konzentrationswert des Wendepunktes unterscheiden, daß das Verfahren zur Berechnung der Konzentration aus den Meßwerten insgesamt zu unbefriedigenden Ergebnissen führt. Wie groß diese Abweichung im Einzelfall sein kann, läßt sich im Einzelfall aufgrund der erfindungsgemäßen Lehre leicht bestimmen. Quantitativ läßt sich sagen, daß der Konzentrationswert des Standardstützpunktes um nicht mehr als 20 %, bevorzugt um nicht mehr als 10 % vom Konzentrationswert des Wendepunktes abweichen sollte.

Weitere Standardstützpunkte befinden sich in der Nähe der Enden des zu bestimmenden Konzentrationsbereiches. Entscheidend ist hier der im praktischen Einsatz tatsächlich benutzte Wertebereich von Konzentrationswerten, nicht der möglicherweise darüberhinaus gehende, zwar technisch mögliche, aber in der Praxis bei dem bestimmten analytischen System nicht benutzte Wertebereich. Der Begriff "in der Nähe" ist analog wie im vorhergehenden Absatz zu verstehen.

Ein wesentliches Merkmal der Erfindung ist weiterhin, daß dem Gerät die chargenspezifischen Auswertekurven nicht in Form einer beispielsweise binär codierten mathematischen Funktion übermittelt werden, die jedem Wert des diffusen Reflektionsvermögens einen Konzentrationswert zuordnen, sondern daß lediglich eine beschränkte Anzahl von diskreten Wertepaaren $R_{ia}$, $C_{ia}$; $R_{ib}$, $C_{ib}$; $R_{ic}$, $C_{ic}$; ... aus einem endlichen Vorrat fest programmierter Berechnungsstützpunkte übermittelt wird. Die Berechnungsstützpunkte sind also im vorhinein unabhängig von der Auswertekurve festgelegt und im Speicher des Auswertegerätes abgespeichert. Je ein Satz von Berechnungsstützpunkten befindet sich jeweils in der Umgebung der Standardstützpunkte. Normalerweise wird in der Umgebung jedes Standardstützpunktes eine gleiche Anzahl Berechnungsstützpunkte definiert, d. h. jeder Satz hat die gleiche Zahl von Punkten. Bei einem Kurvenverlauf der in Figur 2 dargestellten Art mit einem Standardstützpunkt in der Nähe des einzigen Wendepunktes und zwei weiteren Standardstützpunkten in der Nähe der Enden des zu bestimmenden Konzentrationsbereiches, also insgesamt drei Standardstützpunkten, werden also drei gleich große Sätze von Berechnungsstützpunkten definiert. Jeder dieser Sätze kann vorteilhafterweise 16 verschiedene Berechnungsstützpunkte (d.h. die diese Berechnungsstützpunkte definierenden Wertepaare $R_{ia}$, $C_{ia}$; $R_{ib}$, $C_{ib}$; $R_{ic}$, $C_{ic}$; mit jeweils i = 1 ... 16) enthalten. Diese im Gerät programmierten Wertepaare werden dann in der im Zusammenhang mit Figur 1 beschriebenen Art und Weise, beispielsweise mit Hilfe der Tasten 26, 28, 30 in Anpassung auf die jeweiligen Teststreifencharge angewählt. Jedes der 16 mit den Tasten 26, 28, 30 anwählbaren Zeichen entspricht einem Wertepaar. So dient beispielsweise die erste Ziffer der Anwahl von einem der 16 Berechnungsstützpunkte $R_{ia}$, $C_{ia}$, die zweite Ziffer der Anwahl von einem der 16 Berechnungsstützpunkte $R_{ib}$, $C_{ib}$ und die dritte Ziffer der Anwahl von einem der 16 Berechnungsstützpunkten $R_{ic}$, $C_{ic}$. Für jede Testträgercharge erhält der Benutzer des Auswertegerätes eine Anweisung, beispielsweise in Form eines Aufdrucks auf der Verpackung, welche Zeichenfolge er zur Anwahl der Berechnungsstützpunkte auswählen soll. Durch das Eintasten dieser Zeichenfolge wird für jeden Satz von Berechnungsstützpunkten jeweils ein Wertepaar angewählt.

Die Erfindung ist besonders vorteilhaft, wenn die Zahl der zur Auswahl der Wertepaare einzustellenden und zu übermittelnden Informationen klein gehalten wird. Deswegen ist die Zahl der Berechnungsstützpunktsätze, die den Standardstützpunkten auf der Standardkurve zugeordnet werden, auf höchstens zehn, bevorzugt höchstens fünf beschränkt. Für jede Testträgercharge werden somit nur wenige Wertpaare von Berechnungsstützpunkten an den Prozessor übermittelt. Die Zahl der Berechnungsstützpunkte pro Satz beträgt bevorzugt höchstens 256.

Wie erwähnt, liegen die Sätze von Berechnungsstützpunkten in der Umgebung der Standardstützpunkte. Die genauere Lage ergibt sich aus dem weiter unten beschriebenen Iterationsverfahren und kann vom Fachmann aufgrund der erfindungsgemäßen Lehre leicht festgelegt werden.

Bevorzugt liegen die Berechnungsstützpunkte jeweils auf einer die Standardkurve in einem stumpfen Winkel schneidenden Linie. In Figur 2 sind drei derartige Linien a, b und c eingezeichnet. Sie sind den Standardstützpunkten $R_{sa}$, $C_{sa}$; $R_{sb}$, $C_{sb}$ und $R_{sc}$, $C_{sc}$ zugeordnet. In der dargestellten, bevorzugten Ausführungsform schneiden sie die Standardkurve in diesen Punkten. Sie sind bevorzugt, wie dargestellt, näherungsweise gerade ausgebildet.

Figur 3 zeigt nur je einen Teil einer Standardkurve S und einer Auswertekurve I, und zwar den zwischen zwei Standardstützpunkten $R_{sa}$, $C_{sa}$; $R_{sb}$, $C_{sb}$ bzw. zwei Berechnungsstützpunkten $R_{ia}$, $C_{ia}$; $R_{ib}$, $C_{ib}$ liegenden Teil. Bei der hier dargestellten besonders einfachen Ausführungsform der Erfindung liegen die Berechnungsstützpunkte jeweils auf Geraden, die parallel zu einer Koordinate des Koordinatensystems verlaufen. In die Zeichnung sind jeweils zehn den Standardstützpunkten $R_{sa}$, $C_{sa}$ und $R_{sb}$, $C_{sb}$ zugeordnete Berechnungsstützpunkte eingezeichnet. Die dem Standardstützpunkt $R_{sa}$, $C_{sa}$ zugeordneten Berechnungsstützpunkte $R_{1a}$, $C_{1a}$;...; $R_{10a}$, $C_{10a}$ liegen auf der Geraden a, welche parallel zur Ordinate des Koordinatensystems verläuft. Infolgedessen haben alle diese Berechnungsstützpunkte den gleichen Konzentrationswert wie der entsprechende Standardstützpunkt ($C_{1a} = C_{2a} = ... = C_{10a} = C_{sa}$). Dies vereinfacht die Abspeicherung im Gerät, weil für jeden Berechnungsstützpunkt und den Standardstützpunkt nur jeweils der R-Wert getrennt abgespeichert werden muß, während der C-Wert, der für alle Berechnungsstützpunkt und den Standardstützpunkt gleich ist, nur einmal abgespeichert wird. Entsprechendes gilt für die auf den zur Abszisse parallelen Geraden b liegenden Berechnungsstützpunkte $R_{1b}$, $C_{1b}$;...;$R_{10b}$, $C_{10b}$. Hier ist der R-Wert für alle Berechnungsstützpunkte gleich und stimmt mit dem R-Wert des zugehörigen Standardstützpunktes $R_{sb}$ überein.

Das Verfahren zur Berechnung eines Konzentrationswertes $C_{ix}$ aus einem gemessenen Wert der diffusen Reflektion $R_{ix}$ wird im folgenden ebenfalls anhand der Figur 3 erläutert.

Gemessen wird beispielsweise ein diffuses Reflektionsvermögen des Wertes $R_{ix}$. Die wahre Auswertekurve des verwendeten Testträgers ist in Figur 3 gestrichelt dargestellt und mit I bezeichnet. Die durch den Meßwert $R_{ix}$ definierte Horizontale schneidet die Kurve I im Punkt P, der diesem Punkt P zugeordnete Konzentrationswert ist also der wahre Konzentrationswert, der bestimmt werden soll. Ihn exakt zu bestimmen, ist aber nur möglich, wenn die Kurve I für jede Charge im Auswertegerät vollständig abgespeichert ist. Da die Auswertekurven von analytischen Testträgern sich auch mit sehr komplizierten Funktionen höherer Ordnung nicht exakt darstellen lassen, ist dies grundsätzlich nicht möglich. Es wird daher mit Näherungsverfahren gearbeitet, mit Hilfe deren die Auswertekurven näherungsweise dargestellt werden, d. h. jedem Meßwert $R_{ix}$ ein Konzentrationswert $C_{ix}$ zugeordnet wird, der in möglichst guter Näherung der wahren Auswertekurve entspricht. Dies ist grundsätzlich möglich, indem man die jeweilige Auswertekurve der Testträgercharge in der weiter oben bereits angesprochenen Weise in Form einer Funktion höherer Ordnung darstellt und die Parameter dieser Funktion an das Auswertegerät übermittelt. Dies erfordert jedoch einen sehr großen Aufwand, insbesondere bezüglich des Auswertegerätes, wie ebenfalls weiter oben näher erläutert wurde.

Für einfachere Auswertegeräte wird deswegen in der Regel eine einzige mittlere Auswertekurve für alle Testträgerchargen herangezogen. Nimmt man in Figur 3 an, daß die dort eingezeichnete Standardkurve S eine solche mittlere Auswertekurve ist, so sieht man, daß die dem Meßwert $R_{ix}$ entsprechende Horizontale diese Kurve in dem Punkt Q schneidet. Man erkennt sofort, daß der diesem Punkt Q entsprechende Konzentrationswert von dem wahren Konzentrationswert sehr weit abweicht. Eine derartig grobe Annäherung ist deshalb vollkommen unbefriedigend.

Die vorliegende Erfindung erreicht nun mit einem außerordentlich geringen Aufwand eine sehr viel bessere Annäherung an die wahre Auswertekurve, indem aus dem Meßwert $R_{ix}$ zunächst ein Standardmeßwert $R_{sx}$ auf der Standardkurve S und dann aus dem dem Standardmeßwert $R_{sx}$ entsprechenden Konzentrationswert $C_{sx}$ der gesuchte Konzentrationswert $C_{ix}$ berechnet wird. Für den ersten Berechnungsschritt wird einerseits der Abstand der beiden R-Werte, der dem Meßwert $R_{ix}$ nächstliegenden Berechnungsstützpunkte (z. B. $R_{ia}$-$R_{ib}$) und andererseits der Abstand der beiden R-Werte der Standardstützpunkte (z. B. $R_{sa}$-$R_{sb}$), denen diese Berechnungsstützpunkte zugeordnet sind, verwendet. Die Erfindung basiert dabei wesentlich auf der Erkenntnis, daß bei entsprechender Wahl der Berechnungsstützpunkte und der Standardstützpunkte sowie einem im angesprochenen Sinne ähnlichen Kurvenverlauf der Standardkurve zu den Auswertekurven die relative Veränderung eines Remissionswertes gegenüber den benachbarten Berechnungsstützpunktwerten in überraschend guter Näherung ähnlich verläuft, wie die entsprechende Änderung auf der Standardkurve im Vergleich zu den Standardstützpunkten.

Dies läßt sich anhand von Figur 3 näher erläutern, wobei die besonders bevorzugte, aber nicht einzig mögliche lineare Näherung beschrieben wird. Dabei ist die Vorzeichenkonvention des angeführten Berechnungsbeispiels so gewählt, daß sich in dem dargestellten Kurvenabschnitt positive Remissionswerte und Konzentrationswerte ergeben. Für anders geartetee Kurvenabschnitte können die Vorzeichen entsprechend abgewandelt werden.

Zur Berechnung des Standardmeßwertes $R_{sx}$ auf der Standardkurve wird zunächst das Verhältnis $\alpha$ aus dem Abstand des Meßwertes $R_{ix}$ von dem benachbarten Berechnungsstützpunkt $R_{ia}$ zu dem Abstand der Berechnungsstützpunkte $R_{ia}$ und $R_{ib}$ gebildet. Dieses Verhältnis wird mit dem Abstand der Standardstützpunkte $R_{sa}$ und $R_{sb}$ multipliziert und der so erhaltene relative Remissionsabfall gegenüber dem Standardstützpunkt $R_{sa}$ wird von diesem subtrahiert. Das Ergebnis ist der Standardmeßwert $R_{sx}$,

der den dem gemessenen diffusen Reflektionsvermögen $R_{ix}$ im Sinne der vorliegenden Erfindung entsprechenden Wert auf der Standardkurve darstellt.

$$R_{sx} = R_{sa} - \alpha (R_{sa} - R_{sb}); \quad \alpha = \frac{R_{ia} - R_{ix}}{R_{ia} - R_{ib}}$$

Dieser Wert $R_{sx}$ entspricht einem Konzentrationswert $C_{sx}$ auf der Standardkurve S. Dieser Wert wird nun in einem entsprechenden Näherungsverfahren unter Berücksichtigung einerseits des Abstandes der Konzentrationswerte $C_{ia}$, $C_{ib}$ der benachbarten Berechnungsstützpunkte und andererseits der Konzentrationswerte der benachbarten Standardstützpunkte $C_{sa}$, $C_{sb}$ umgerechnet. In linearer Näherung wird die Relation zwischen dem Abstand des Wertes $C_{sx}$ von dem benachbarten Standardkonzentrationswert $C_{sa}$ zu dem Abstand zwischen den Standardkonzentrationswerten $C_{sa}$ und $C_{sb}$ gebildet. Dieses Berhältnis β wird mit dem Abstand der beiden Konzentrationswerte der Verechnungsstützpunkte $C_{ib}$ und $C_{ia}$ multipliziert und zu dem Konzentrationswert $C_{ia}$ des benachbarten Berechnungsstützpunktes addiert.

$$C_{ix} = \beta (C_{ib} - C_{ia}) + C_{ia}; \quad \beta = \frac{C_{sx} - C_{sa}}{C_{sb} - C_{sa}}$$

Auf diese Weise wird der Konzentrationswert $C_{ix}$ erhalten. Dieser entspricht nicht exakt, aber in sehr guter Näherung dem zu messenden Konzentrationswert. Dies wird in Figur 3 dadurch verdeutlicht, daß der Punkt $C_{ix}$, $R_{ix}$ von dem auf der wahren Auswertekurve liegende Punkt P geringfügig abweicht.

Wie erwähnt, kann statt der im einzelnen beschriebenen linearen Näherung auch ein anderes Näherungsverfahren gewählt werden, bei dem die Relation der erwähnten Abstände zwischen des Berechnungsstützpunkten einerseits und den Standardstützpunkten andererseits berücksichtigt wird. In besonderen Fällen kann sich beispielsweise eine logarithmische oder quadratische Näherung empfehlen. Die beschriebene lineare Näherung ist jedoch besonders einfach und führt zu sehr befriedigenden praktischen Ergebnissen.

Die Qualität der Näherung gemäß dem erfindungsgemäßen Verfahren ist sehr wesentlich von der Wahl der Standardkurve, der Standardstützpunkte und der Berechnungsstützpunkte innerhalb der im einzelnen dargelegten und beanspruchten Grenzen abhängig. Zur Optimierung empfiehlt sich ein Iterationsverfahren, das etwa wie folgt abläuft:

Zunächst wird eine Vielzahl von Testträgerchargen hergestellt und die zugehörigen Auswertekurven werden exakt vermessen. Danach wird eine erste Standardkurve so gelegt, daß sie etwa in der Mitte der Auswertekurven verläuft. Die Standardstützpunkte werden zunächst genau auf die Wendepunkte der Standardkurve und auf die Enden des Meßbereichs gelegt. Durch die Standardstützpunkte werden gerade oder nur leicht gekrümmte in einem stumpfen Winkel zum Verlauf der Standardkurve in dem jeweiligen Standardstützpunkt verlaufende Kurven gelegt. Bevorzugt sollte man der Einfachheit halber zunächst Geraden versuchen, die parallel zu den Achsen des Koordinatensystems verlaufen. Auf diesen werden Berechnungsstützpunkte definiert, die gleiche Abstände voneinander haben. Die Abstände und die Verteilung der Berechnungsstützpunkte auf beiden Seiten des Standardstützpunktes werden dabei so gewählt, daß die äußersten Berechnungsstützpunkte noch außerhalb der äußersten Auswertekurven der untersuchten Testträgerchargen liegen.

Für jede Testträgercharge werden nun zunächst diejenigen Berechnungsstützpunkte aus jedem Satz von Berechnungsstützpunkten gewählt, die möglichst nahe bei dem Kurvenverlauf liegen. Mit Hilfe dieser Berechnungsstützpunkte und des erfindungsgemäßen Verfahrens wird der angenäherte Verlauf der Auswertekurve berechnet, graphisch dargestellt und mit der jeweiligen tatsächlichen Auswertekurve verglichen. Werden alle zuvor vermessenen Auswertekurven mit der gewünschten Qualität der Näherung richtig dargestellt, so ist das Verfahren beendet. Im Regelfall werden aber zumindest einige Auswertekurven, zumindest in Teilbereichen, nicht mit der notwendigen Qualität wiedergegeben. In derartigen Fällen wird der Verlauf der Standardkurve und/oder die Wahl der Standardstützpunkte und der Berechnungsstützpunkte verändert und ein neuer Probelauf durchgeführt, um zu prüfen, ob nunmehr die notwendige Genauigkeit erreicht ist. Dieses Verfahren erscheint mühsam, kann jedoch mit modernen Datenverarbeitungseinrichtungen verhältnismäßig schnell routinemäßig durchgeführt werden. Entscheidend ist, daß aufgrund der grundlegenden in den Ansprüchen definierten Lehren der vorliegenden Erfindung auch sehr kompliziert gestaltete Auswertekurven mit einem äußerst geringen Aufwand seitens des Auswertegerätes und mit sehr guter Genauigkeit berechnet werden können, d. h. daß das letztlich möglich ist, chargenspezifische Abweichungen der Testträger untereinander in einem verhältnismäßig einfach aufgebauten und kostengünstigen Gerät zu berücksichtigen, wobei zugleich das System aus Gerät und Testträger besonders einfach zu handhaben ist.

Die Erfindung kann vorteilhaft auch für Auswertegeräte eingesetzt werden, bei denen mehrere verschiedene Typen von Testträgern vermessen werden können, d. h. mit denen die Bestimmung mehrerer verschiedener Parameter möglich ist. In diesem Fall können für jeden Testträgertyp getrennt die Standardkurve, die Standardstützpunkte und die Berechnungsstützpunkte abgespeichert werden. Je nach dem Kurvenverlauf ist es jedoch auch möglich, daß die gleichen Berechnungsstützpunkte oder Standardstützpunkte und unter Umständen auch Teile des Verlaufs der Standardkurven für mehrere Testträgertypen gemeinsam benutzt werden. Dadurch wird Speicherkapazität gespart. Selbstverständlich muß für jede Testträgercharge eines bestimmten Testträgertyps die jeweilige zugeordnete Kombination der Berechnungsstützpunkte getrennt angewählt werden. Wenn beispielsweise für jeden Testträgertyp drei Sätze von Berechnungsstützpunkten zur Verfügung stehen, kann die Anwahl zweckmäßigerweise durch eine Folge von vier Zeichen erfolgen. Das erste Zeichen charakterisiert dann den Testträgertyp. Die weiteren drei Zeichen charakterisieren die Auswahl der Berechnungsstützpunkte aus dem endlichen Vorrat der für den jeweiligen Testträgertyp abgespeicherten Berechnungsstützpunkte.

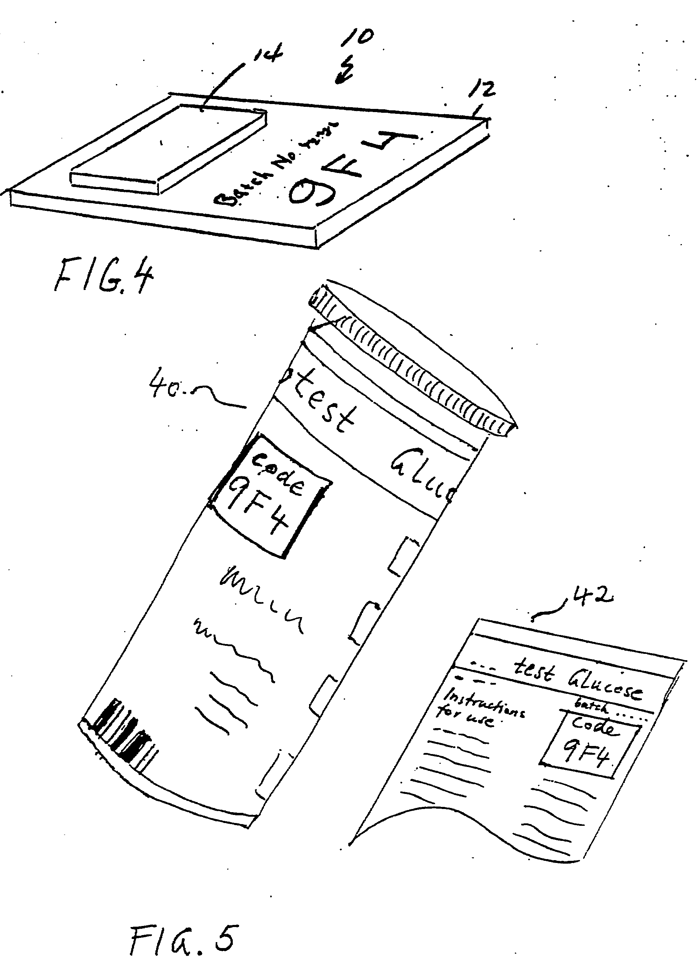

Fig. 4 zeigt beispielhaft, wie die sichtbaren Zeichen, aufgrund deren die Berechnungsstützpunkte ausgewählt werden, auf einem Testträger 10 angebracht sind. In diesem Fall sind es die alphanumerischen Zeichen "9F4", aufgrund deren drei Berechnungsstützpunkte aus einem Satz von jeweils 16 Berechnungsstützpunkten ausgewählt werden. Sie sind auf der Basis 12 des Testträgers 10 aufgebracht. Alle Testträger einer Herstellungscharge haben die gleichen sichtbaren Zeichen.

Fig. 5 zeigt ein Beispiel, bei dem die sichtbaren Zeichen auf einer Testträgerverpackung 40 und/oder einer entsprechenden Packungsbeilage 42 aufgebracht sind. Bei diesem Verfahren wird die Herstellung vereinfacht, weil nicht jeder Testträger mit dem sichtbaren Zeichen bedruckt werden muß.

**Patentansprüche**

1. System zur Bestimmung der Konzentration (C) von Bestandteilen von Körperflüssigkeiten umfassend

(1) einen Testträger (10) mit einem Testfeld (14), welches ein Reagenz enthält, das nach Aufbringen der Probenflüssigkeit mit dem Bestandteil reagiert, wobei sich eine meßbare physikalische Größe, insbesondere das diffuse Reflexionsvermögen des Testfeldes (14), in Abhängigkeit der Konzentration (C) des Bestandteils der Körperflüssigkeit ändert und

(2) ein Gerät mit einer Meßeinrichtung (18) zur Messung der physikalischen Größe und einer einen Speicher und einen Prozessor (20) einschließenden Auswerteelektronik zur Messung des Meßwerts (R) der physikalischen Größe und Berechnung der Konzentration (C) aus dem Meßwert (R) aufgrund von dem Gerät übermittelten Informationen über die Auswertekurve, d.h. der Relation zwischen dem Meßwert (R) der physikalischen Größe und der Konzentration (C), welche in Abhängigkeit von der Herstellungscharge des Testträgers unterschiedlich ist, dadurch gekennzeichnet, daß

a) in dem Gerät eine endliche Zahl diskreter Berechnungsstützpunkte gespeichert ist, wobei jeder Berechnungsstützpunkt einem Wertepaar aus je einem R-Wert und je einem C-Wert ($R_{ia}$, $C_{ia}$; $R_{ib}$, $C_{ib}$; ...) entspricht

b) an den Testträgern, deren Verpackung oder einer Packungsbeilage eine Mehrzahl sichtbarer Zeichen angebracht ist, die der jeweiligen Herstellungscharge der Testträger zugeordnet sind,

c) das Gerät Einrichtungen (26, 28, 30) zur manuellen Eingabe der sichtbaren Zeichen aufweist,

d) in dem Gerät aufgrund der Eingabe bestimmte Berechnungsstützpunkte aus der endlichen Zahl ausgewählt werden, welche für die tatsächliche Relation zwischen dem Messwert (R) und der Konzentration (C) bei der jeweiligen Herstellungscharge repräsentativ sind und

e) der Prozessor (20) die dem jeweiligen Meßwert (R) entsprechende Konzentration in einem Näherungsverfahren mit Hilfe der ausgewählten Berechnungsstützpunkte berechnet.

2. System nach Anspruch 1, dadurch gekennzeichnet, daß

a) in dem Speicher eine Standardauswertekurve gespeichert ist, deren Verlauf dem der Auswertekurven (I, J) der von dem Gerät auszuwertenden Testträger in dem Sinne ähnlich ist,

aa) daß sie im Wertebereich der Auswertekurven (I, J) verläuft und

ab) daß sie eine konvexe bzw. konkave Krümmung im wesentlichen in den gleichen Abschnitten von Konzentrationswerten aufweist wie die Auswertekurve;

b) auf der Standardkurve in der Nähe des Wendepunktes zwischen einem konkav- und einem konvex gekrümmten Abschnitt und in der Nähe der Enden des zu bestimmenden Konzentrationsbereiches ein Standardstützpunkt aus je einem R-Wert und je einem C-Wert ($R_{sa}$, $C_{sa}$; $R_{sb}$, $C_{sb}$; ...) definiert ist;

c) jedem Standardstützpunkt (z.B. $R_{sa}$, $C_{sa}$) ein bestimmter Satz von Berechnungsstützpunkten (z.B. $R_{ia}$, $C_{ia}$ mit i = 1 ... 10) zugeordnet ist, der in der Umgebung des Standardstützpunktes liegt;

d) das Näherungsverfahren zweistufig abläuft, wobei

da) zuerst der jeweils gemessene Meßwert ($R_{ix}$) in einen Standardmeßwert ($R_{sx}$) auf der Standard-

kurve (S) unter Berücksichtigung der relativen Größe des Abstandes der beiden R-Werte ($R_{ia}$, $R_{ib}$) der nächstliegenden ausgewählten Berechnungsstützpunkte einerseits und des Abstandes der beiden R-Werte ($R_{sa}$, $R_{sb}$) der Standardstützpunkte, denen diese Berechnungsstützpunkte zugeordnet sind, andererseits umgerechnet wird, und

db) danach der dem Standardmeßwert ($R_{sx}$)) entsprechende Konzentrationswert ($C_{sx}$)) auf der Standardkurve (S) unter Berücksichtigung der relativen Größe des Abstands der beiden C-Werte ($C_{ia}$, $C_{ib}$) der nächstliegenden Berechnungsstützpunkte einerseits und des Abstands der beiden C-Werte ($C_{sa}$, $C_{sb}$) der nächstliegenden Standardstützpunkte, denen die Berechnungsstützpunkte zugeordnet sind, andererseits in den gesuchten Konzentrationswert ($C_{ix}$) umgerechnet wird.

3. System nach Anspruch 2, **dadurch gekennzeichnet, daß**
der Standardmeßwert $R_{sx}$ (Merkmal c von Anspruch 1) in linearer Näherung berechnet wird,

$$R_{sx} = R_{sa} - \alpha \, (R_{sa} - R_{sb}); \ \alpha = \frac{R_{ia} - R_{ix}}{R_{ia} - R_{ib}}$$

und daß die gesuchte Konzentration ebenfalls in linearer Näherung berechnet wird

$$C_{ix} = \beta \, (C_{ib} - C_{ia}) + C_{ia}; \ \beta = \frac{C_{sx} - C_{sa}}{C_{sb} - C_{sa}}$$

4. System nach einem der Ansprüche 2 oder 3, **dadurch gekennzeichnet, daß**
die einem Satz zugehörigen Berechnungsstützpunkte (z.B. $R_{ia}$, $C_{ia}$ mit i = 1 ...10) in der Umgebung eines Standardstützpunktes ($R_{sa}$, $C_{sa}$) auf einer die Standardkurve in einem stumpfen Winkel schneidenden näherungsweise geraden Linie (a,b,c) liegen.

5. System nach einem der Ansprüche 2 oder 3, **dadurch gekennzeichnet, daß**
die einem Satz zugehörigen Berechnungsstützpunkte (z.B. $R_{ia}$, $C_{ia}$ mit i = 1...10) jeweils auf einer Geraden liegen, die parallel zu einer Koordinate des Koordinatensystems R-C verläuft.

6. System nach einem der Ansprüche 1 bis 5, **dadurch gekennzeichnet, daß**
mindestens zwei und höchstens fünf Sätze von Berechnungsstützpunkten vorgesehen sind.

7. System nach Anspruch 2, **dadurch gekennzeichnet, daß**
die aufgrund der sichtbaren Zeichen ausgewählten Berechnungsstützpunkte innerhalb eines Satzes von Berechnungsstützpunkten diejenigen sind, die dem tatsächlichen Verlauf der Auswertekurve der jeweiligen Herstellungscharge am nächsten oder zweitnächsten liegen.

8. System nach Anspruch 1, **dadurch gekennzeichnet, daß**
die sichtbaren Zeichen alphanumerische Zeichen sind.

9. System nach Anspruch 1, **dadurch gekennzeichnet, daß**
höchstens fünf alphanumerische Zeichen vorgesehen sind.

## Claims

1. System for the determination of the concentration (C) of components of body fluids comprising
(1) a test carrier (10) with a test field (14) which contains a reagent which, after application of the sample liquid, reacts with the component, whereby a measurable physical value, especially the diffuse reflectance of the test field (14), changes depending upon the concentration (C) of the component of the body fluid and
(2) an apparatus with a measurement device (18) for the measurement of the physical value and a memory and a processor (20) including evaluation electronics for the measurement of the test value (R) of the physical value and calculation of the concentration (C) from the test value (R) on the basis of information transmitted to the apparatus via the evaluation curve, i.e. the relation between the test value (R) of the physical value and the concentration (C) which is different depending upon the production batch of the test carrier, characterised in that
a) in the apparatus is stored a finite number of discrete calculation bearings, whereby each calculation bearing corresponds to a pair of values of, in each case, an R value and, in each case, a C value ($R_{ia}$, $C_{ia}$; $R_{ib}$, $C_{ib}$; .....)
b) on the test carriers, their packing or a packing enclosure is applied a plurality of visible marks which are associated with the particular production batch of the test carrier,
c) the apparatus has equipment (26, 28, 30) for the manual application of the visible marks,

EP 0 247 439 B1

d) in the apparatus, on the basis of the application, certain calculation bearings are selected from the finite number which are representative for the actual relation between the test value (R) and the concentration in the case of the particular production batch and e) the processor (20) calculates the concentration corresponding to the particular test value (R) in an approximation process with the help of the selected calculation bearings.

2. System according to claim 1, characterised in that
a) in the memory is stored a standard evaluation curve the course of which is similar to the evaluation curves (I, J) of the test carrier to be evaluated by the apparatus in the sense
    aa) that it runs in the value range of the evaluation curves (I, J) and
    ab) that it displays a convex or concave bend essentially in the same section of concentration values as the evaluation curve;
b) on the standard curve in the region of the cusp between a concave and a convex bent section and in the region of the ends of the concentration range to be determined, a standard bearing is defined from, in each case, an R value and, in each case, a C value ($R_{sa}$, $C_{sa}$; $R_{sb}$, $C_{sb}$; .....);
c) with each standard bearing (e.g. $R_{sa}$, $C_{sa}$) is associated a set of calculation bearings (e.g. $R_{ia}$, $C_{ia}$ with i = 1 .... 10) which lie in the region of the standard bearing;
d) the approximation process takes place in two steps, whereby
    da) first, the particular measured test value ($R_{ix}$) is converted into a standard test value ($R_{sx}$) on the standard curve (S) having regard to the relative size of the distance of the two R values ($R_{ia}$, $R_{ib}$) of the adjacent selected calculation bearings on the one hand and to the distance of the two R-values ($R_{sa}$, $R_{sb}$) of the standard bearings which are associated with these calculation bearings on the other hand, and
    db) thereafter, the concentration value ($C_{sx}$) corresponding to the standard test value ($R_{sx}$) is converted into the desired concentration value ($C_{ix}$) having regard to the relative size of the distance of the two C values ($C_{ia}$, $C_{ib}$) of the adjacent calculation bearings on the one hand and of the distance of the two C values ($C_{sa}$, $C_{sb}$) of the adjacent standard bearings with which these calculation bearings are associated on the other hand.

3. System according to claim 2, characterised in that the standard test value $R_{sx}$ (feature C of claim 1) is calculated in linear approximation

$$R_{sx} = R_{sa} - \alpha (R_{sa} - R_{sb}); \quad \alpha = \frac{R_{ia} - R_{ix}}{R_{ia} - R_{ib}}$$

and that the concentration sought is also calculated in linear approximation

$$C_{ix} = \beta (C_{ib} - C_{ia}) + C_{ia}; \quad \beta = \frac{C_{sx} - C_{sa}}{C_{sb} - C_{sa}}$$

4. System according to one of claims 2 or 3, characterised in that the calculation bearings belonging to one set (e.g. $R_{ia}$, $C_{ia}$ with i = 1 .... 10) lies in the region of a standard bearing ($R_{sa}$, $C_{sa}$) on an approximately straight line (a, b, c) cutting the standard curve in an obtuse angle.

5. System according to one of claims 2 or 3, characterised in that the calculation bearings (e.g. $R_{ia}$, $C_{ia}$ with i = 1 ... 10) belonging to a set each lie on a line which runs parallel to a coordinate of the coordinate system R – C.

6. System according to one of claims 1 to 5, characterised in that at least two and at most five sets of calculation bearings are provided.

7. System according to claim 2, characterised in that the calculation bearings selected on the basis of the visible marks within a set of calculation bearings are those which lie next to or second next to the actual course of the evaluation curve of the particular production batch.

8. System according to claim 1, characterised in that the visible marks are alphanumeric marks.

9. System according to claim 1, characterised in that at most five alphanumeric marks are provided.

10

**Revendications**

1. Système pour déterminer la concentration (C) de constituants de fluides corporels, comprenant:
(1) un porte-test (10) comportant un champ de test (14), qui contient un réactif, qui réagit avec le constituant, étant entendu qu'une grandeur mesurable physiquement, en particulier le pouvoir de réflexion diffuse du champ de test (14), varie en fonction de la concentration (C) du constituant du fluide corporel, et
(2) un appareil comportant un dispositif de mesure (18) de la grandeur physique et une électronique d'évaluation comprenant une mémoire et un processeur (20) pour déterminer la valeur de mesure (R) de la grandeur physique et calculer la concentration (C) à partir de la valeur de mesure (R), en se basant sur des informations provenant de l'appareil au moyen de la courbe d'évaluation, c'est-à-dire au moyen de la relation entre la valeur de mesure (R) de la grandeur physique et la concentration (C), laquelle est variable en fonction de la charge de préparation du porte-test, caractérisé en ce que
a) dans l'appareil, un nombre final des points de base de calcul est enregistré, chaque point de base de calcul correspondant à un couple de valeurs formé chacun d'une valeur R et d'une valeur C ($R_{ia}$, $C_{ia}$; $R_{ib}$, $C_{ib}$; ...),
b) sur le porte-test, sur l'emballage de celui-ci, ou sur un accessoire d'emballage de celui-ci, sont reportés plusieurs signes visibles, qui sont associés à chacune des charge de préparation du porte-test,
c) l'appareil présente des dispositifs (26, 28, 30) pour introduire manuellement les signes visibles,
d) dans l'appareil, des points de base de calcul déterminés suite à l'introduction faite, et qui sont représentatifs, pour chacune des charges de préparation, de la relation réelle entre la valeur de mesure (R) et la concentration (C), sont choisis à partir du nombre final, et
e) le processeur (20) calcule, suivant une méthode d'approximation, à l'aide des points de base de calcul. la concentration correspondant à chacune des valeurs de mesure (R).
2. Système suivant la revendication 1. caractérisé en ce que
a) dans la mémoire, est stockée une courbe standard d'évaluation, dont le tracé est analogue à celui des courbes d'évaluation (I, J) des porte-test à évaluer par l'appareil,
aa) en ce qu'elle est comprise dans le domaine de valeur des courbes d'évaluation (I, J),
ab) en ce qu'elle présente, comme les courbes d'évaluation, une courbure concave ou convexe essentiellement dans les même plages des valeurs de la concentration;
b) sur la courbe standard, au voisinage du point d'inflexion entre une plage de courbure concave et une plage de courbure convexe, et au voisinage des extrémités de la zone de la concentration à déterminer, un point de base standard est défini à partir de chacune des valeurs R et de chacune des valeurs C ($R_{sa}$, $C_{sa}$; $R_{sb}$, $C_{sb}$; ...);
c) à chaque point de base standard (par exemple $R_{sa}$, $C_{sa}$), est associé un groupe déterminé de points de base de calcul (par exemple $R_{ia}$, $C_{ia}$ avec i = 1 ... 10), qui se trouve au voisinage du point de base standard;
d) le procédé d'approximation se déroule en deux étapes,
da) d'abord chacune des valeurs de mesure ($R_{ix}$) est recalculée en une valeur de mesure standard ($R_{sx}$) sur la courbe standard (S), en prenant en compte le rapport des grandeurs de l'écart des deux valeurs R ($R_{ia}$, $R_{ib}$) des points de base de calcul successifs choisis, d'une part, et de l'écart des deux valeurs R ($R_{sa}$, $R_{sb}$) des points de base standards, auxquels ces points de base de calcul sont associés. d'autre part, et
db) ensuite, la valeur de la concentration ($C_{sx}$), correspondant à la valeur de mesure standard ($R_{sx}$), est recalculée en la valeur de la concentration cherchée ($C_{ix}$), sur la courbe standard (S), en prenant en compte le rapport des grandeurs de l'écart des deux valeurs C ($C_{ia}$, $C_{ib}$) des points de base de calcul suivants, d'une part, et de l'écart des deux valeurs C ($C_{sa}$, $C_{sb}$) des points de base standards successifs, auxquels les points de base de calcul sont associés, d'autre part.
3. Système suivant la revendication 1, caractérisé en ce que la valeur de mesure standard Rsx (partie caractérisante c de la revendication 1) est calculée en approximation linéaire:

$$R_{sx} = R_{sa} - \alpha\,(R_{sa} - R_{sb}); \quad \alpha = \frac{R_{ia} - R_{ix}}{R_{ia} - R_{ib}}$$

et en ce que la concentration cherchée est calculée de même en approximation linéaire:

$$C_{ix} = \beta \, (C_{ib} - C_{ia}) + C_{ia}; \quad \beta = \frac{C_{sx} - C_{sa}}{C_{sb} - C_{sa}}$$

4. Système suivant l'une des revendications 2 ou 3, caractérisé en ce que les points de base de calcul correspondant à un groupe de valeurs (par exemple $R_{ia}$, $C_{ia}$ avec $i = 1...10$) se trouvent, au voisinage d'un point de base standard ($R_{sa}$, $C_{sa}$), sur une ligne approximativement droite (a, b, c), coupant sous un angle obtus la courbe standard.

5. Système suivant l'une des revendications 2 ou 3, caractérisé en ce que chacun des points de base de calcul correspondant à un groupe de valeurs (par exemple $R_{ia}$, $C_{ia}$ avec $i = 1...10$) se trouve sur une droite disposée parallèlement à un axe de coordonnée du système de coordonnées R-C.

6. Système suivant l'une quelconque des revendications 1 à 5, caractérisé en ce qu'au moins deux et, au plus, cinq groupes de valeurs de points de base de calcul sont prévus.

7. Système suivant la revendication 2. caractérisé en ce que les points de base de calcul choisis sur la base des signes visibles à l'intérieur d'un groupe de points de base de calcul, sont ceux qui se suivent, ou se trouvent être les deuxièmes en suivant, sur le tracé réel de la courbe d'évaluation de chaque charges de préparation concernée.

8. Système suivant la revendication 1, caractérisé en ce que les signes visibles sont des signes alphanumériques.

9. Système suivant la revendication 1, caractérisé en ce qu'au plus, cinq signes alphanumériques sont prévus.

Fig.1

EP 0 247 439 B1

Fig.2

Fig.3

FIG.4

FIG.5